# EUROPEAN PATENT SPECIFICATION

(11) **EP 2 366 024 B1**
(45) Date of publication and mention of the grant of the patent: **01.05.2019**
(21) Application number: 09831233.3
(22) Date of filing: 04.12.2009
(51) Int. Cl.: C12N 7/00

(54) **COMPOSITIONS, METHODS AND USES FOR INDUCING VIRAL GROWTH**
ZUSAMMENSETZUNGEN, VERFAHREN UND VERWENDUNG ZUR EINLEITUNG VON VIRENWACHSTUM
COMPOSITIONS, PROCÉDÉS ET UTILISATIONS POUR INDUIRE LA CROISSANCE VIRALE

(30) Priority: 05.12.2008 US 120262 P
(43) Date of publication of application: 21.09.2011
(73) Proprietor: Takeda Vaccines, Inc., Cambridge, MA 02139 (US)
(72) Inventor: STINCHCOMB, Dan, T., Fort Collins Colorado 80528 (US); LIVENGOOD, Jill, A., Fort Collins Colorado 80526 (US); WIGGAN, O'Neil, Fort Collins Colorado 80521 (US); KINNEY, Richard, Fort Collins Colorado 80526 (US); OSORIO, Jorge, Mount Horeb Wisconsin 53572 (US)
(74) Representative: Huenges, Martin
(86) International application number: PCT/US2009/066848
(87) International publication number: WO 2010/065911

(56) References cited:
- WO-A2-03/084479
- US-A- 5 616 487
- US-A1- 2006 148 074
- US-A1- 2008 050 770
- US-A1- 2008 248 551
- XIE LIANGZHI ET AL: "Large-scale propagation of a replication-defective adenovirus vector in stirred-tank bioreactor PER.C6TM cell culture under sparging conditions", BIOTECHNOLOGY AND BIOENGINEERING, WILEY & SONS, HOBOKEN, NJ, US, vol. 83, no. 1, 5 July 2003 (2003-07-05), pages 45-52, XP002296365, ISSN: 0006-3592, DOI: 10.1002/BIT.10644
- KHATTAK SARWAT F ET AL: "Pluronic F127 as a cell encapsulation material: utilization of membrane-stabilizing agents.", TISSUE ENGINEERING 2005 MAY-JUN LNKD- PUBMED:15998236, vol. 11, no. 5-6, May 2005 (2005-05), pages 974-983, XP002676374, ISSN: 1076-3279

## Description

### Field

The present invention is related to a composition for growing viral cultures, a method for increasing viral growth rate, a method for increasing plaque size of a viral culture, a method for reducing growth lag time of a viral culture, and a kit for culturing viruses.

### Background

Vaccines to protect against infectious diseases have been used to improve human and animal health. One successful technology for viral vaccines is to immunize animals or humans with a weakened or attenuated strain of the virus (a "live, attenuated virus"). Due to limited replication after immunization, the attenuated strain does not cause disease. However, the limited viral replication is sufficient to express the full repertoire of viral antigens and generates potent and long-lasting immune responses to the virus. Thus, upon subsequent exposure to a pathogenic strain of the virus, the immunized individual is protected from disease.

Recent technical advances, such as reassortment, reverse genetics and cold adaptation, have led to advances of live, attenuated viruses for influenza and rotavirus. A number of live, viral vaccines developed with recombinant DNA technologies are in human clinical testing, including vaccines for West Nile disease, dengue fever, malaria, tuberculosis and HIV. These recombinant viral vaccines rely on manipulation of well-characterized attenuated viral vaccines, such as adenovirus, vaccinia virus, yellow fever 17D or the dengue virus, DEN-2 PDK-53. As a group, live attenuated viral vaccines are amongst the most successful medical interventions in human history, second only to the advent of antibiotics and hold the promise to improve public health throughout the world.

Other vaccines have been developed by inactivating viruses after growth in cell culture. These "killed virus" vaccines induce immune responses due to the presence of high concentrations of antigen present. Examples of effective killed viral vaccines include, but are not limited to, vaccine for rabies, influenza, hepatitis A, and poliovirus.

Flaviviruses cause a number of human and animal diseases of significant impact. They are enveloped viruses with a RNA genome of approximately 11,000 bases. Most of the flaviviruses are transmitted by an arthropod vector, commonly mosquitoes. There are over 70 different flaviviruses that are grouped into three major categories based on serology: the dengue group, the Japanese encephalitis group and the yellow fever group. Expanding urbanization, worldwide travel and environmental changes (such as deforestation or rain patterns) have lead to the emergence of several flaviviruses as threats to human public health. Such viruses include, but are not limited to, yellow fever virus, the dengue viruses, West Nile virus, Japanese encephalitis virus, and tick-borne encephalitis viruses.

Both live, attenuated viral vaccines and killed virus vaccines have been developed that are safe and protect against flavivirus diseases, for example, yellow fever and Japanese encep halitis.

In an embodiment of the first aspect, the viral culture comprises flavivirus and the host cell culture comprises Vero (African green monkey Vero cells), LLC-MK2 cells (monkey kidney cells) or C6/36 mosquito cells.

More specifically, the problem underlying the present invention is solved in a second aspect by a method for increasing viral growth rate comprising, administering to a host cell culture infected with a virus, a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), and wherein the composition increases viral growth rate.

More specifically, the problem underlying the present invention is solved in a third aspect by a method for increasing viral growth rate comprising, administering a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), to a host cell culture, before, during, or after viral infection of the host cell culture.

More specifically, the problem underlying the present invention is solved in a fourth aspect by a method for increasing plaque size of a viral culture comprising, administering to a host cell culture infected with a virus, a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), and wherein the composition increases viral plaque size compared to a control viral culture without administering one or more EO-PO copolymers.

US 2008/0050770 A1 is related to a method for the production and purification of adenoviral vectors.

US 2008/0248551 A1 is related to methods and compositions for live attenuated viruses.

US 2006/0148074 is related to a serum-free mammalian cell culture medium, and uses thereof.

Xie L et al. (Xie L et al., Biotechnology and Bioengineering, vol. 83, no. 1, July 5, 2003, pp. 45-52) report on large-scale propagation of a replication-defective adenovirus vector in stirred-tank bioreactor PER.C6™ cell culture under sparging conditions.

WO 03/084479 A2 is related to large-scale methods of producing adenovirus and adenovirus seed stocks.

US 5,616,487 is related to stabilized retrovirus compositions.

Khattak SF et al. (Khattak SF et al., Tissue Engineering, vol. 11, no. 5/6, 2005, pp. 974-983) report on Pluronic F127 as a cell encapsulation material and utilization of membrane-stabilizing agents.

### Summary

The problem underlying the present invention is solved by the subject matter of the attached independent claims. Preferred embodiments may be taken from the attached dependent claims.

More specifically, the problem underlying the present invention is solved in a first aspect by a composition for growing viral cultures comprising:
one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v);
a viral culture;
a host cell; and
a medium for growing the viral culture,
wherein the EO-PO block copolymer accelerates growth of viral cultures.

In an embodiment of the first aspect, the viral cultures are selected from the group consisting of Flavivirus, Togavirus, Coronavirus, Rhabdovirus, Filovirus, Paramyxovirus, Orthomyxovirus, Bunyavirus, Arenavirus, Retrovirus, Hepadnavirus, Pestivirus, Picornavirus, Calicivirus, Reovirus, Parvovirus, Papovavirus, Adenovirus, Herpes virus, and Poxvirus.

In an embodiment of the first aspect, the viral cultures are flavivirus cultures or poxvirus cultures.

In an embodiment of the first aspect, the viral cultures are dengue virus cultures.

In an embodiment of the first aspect, at least one of the EO-PO block copolymer is poloxamer 407 (Pluronic F-127) and its concentration is about 0.063 % to about 2.0 % (w/v).

In an embodiment of the first aspect, the medium comprises Dulbecco's Modified Eagle Medium (DMEM).

More specifically, the problem underlying the present invention is solved in a fifth aspect by a method for reducing growth lag time of a viral culture comprising, administering to a host cell culture infected with a virus, a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), and wherein the composition reduces lag time of the viral cultures compared to a control viral culture without administering one or more EO-PO block copolymers.

In an embodiment of the fifth aspect, the viral cultures are flaviviral cultures.

More specifically, the problem underlying the present invention is solved in a sixth aspect by a kit for culturing viruses comprising;
at least one container;
a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v); and
one or more viral cultures,
wherein the EO-PO block copolymer accelerates growth of viral cultures.

In an embodiment of the sixth aspect, the EO-PO block copolymer is poloxamer 407 (Pluronic F-127) and the concentration of the EO-PO block copolymer is about 0.063 % to about 2.0 % (w/v).

In an embodiment of the sixth aspect, the one or more viral cultures comprise one or more flavivirus cultures.

In an embodiment of the sixth aspect, the one or more viral cultures comprise one or more dengue virus cultures.

One limitation for producing vaccines has been large-scale manufacture and in vitro growth of the viruses to support the demand of vaccines. Thus, one of the needs that exist in the art is for enhancing and accelerating viral growth. Certain embodiments of the present invention as defined in the claims concern methods and compositions for enhancing and accelerating viral growth. These compositions are of use, for example, in production of viral vaccines and viral byproducts of use in other technologies such as manufacturing of viral-related gene therapies and other viral products. In addition, embodiments herein may be of use to enhance or accelerate growth of viral cultures of use in killed virus vaccines.

Certain compositions disclosed herein can include copolymers alone or in combination with other agents or compounds for enhancing and accelerating viral growth. Other compositions concern combinations of excipients that enhance growth of live attenuated viruses. Copolymers of use herein include, but are not limited to, Pluronic F127, Pluronic F68, Pluronic P123, Pluronic P85, other polyethylene oxide-polypropylene oxide (EO-PO) block copolymers of greater than 3,000-4,000 MW or combinations thereof.

In accordance with embodiments of the invention as defined in the claims, viruses can include, but are not limited to, Flavivirus, Togavirus, Coronavirus, Rhabdovirus, Filovirus, Paramyxovirus, Orthomyxovirus, Bunyavirus, Arenavirus, Retrovirus, Hepadnavirus, Pestivirus, Picornavirus, Calicivirus, Reovirus, Parvovirus, Papovavirus, Adenovirus, Herpes virus, and Poxvirus. Some embodiments of the invention as defined in the claims can include, but are not limited to, cultures having one or more viruses, such as a mixture of viral species or a single species, or one or more live, attenuated viruses grown in one or more copolymer compositions alone, or in combination with other agents.

In other embodiments of the invention as defined in the claims, compositions contemplated herein can increase plaque size in a reduced or similar time period of growth, compared to a control culture without the disclosed composition, for use in tittering, manufacturing or measuring the activity of virus preparations. In some aspects of the present invention as defined in the claims higher viral titers may be obtained in reduced time periods. Alternatively, composition of the invention as defined in the claims contemplated herein can reduce lag time or accelerate growth time up to several days compared to control viral cultures not using compositions contemplated herein.

Disclosed are virus populations for use in formulations and methods directed to vaccine formulations capable of reducing or preventing onset of a medical condition caused by one or more of the viruses contemplated herein. In accordance therewith medical conditions may include, but are not limited to conditions and/or infections including West Nile, dengue fever, Japanese encephalitis, Kyasanur forest disease, Murray valley encephalitis in Australia and New Guinea, Kunjin virus (a relative of West Nile), Alkhurma hemorrhagic fever, St. Louis encephalitis, hepatitis C virus infection, tick-borne encephalitis, yellow fever, the Usutu, Koutango, Yaondeviruses in Africa, and Cacipacore in South America. In certain embodiments of the invention as defined in the claims production time for generating vaccine formulations may be reduced by using compositions contemplated herein for accelerating viral growth production and manufacture, reducing lag time and/or increasing plague size of viral populations.

Viral cultures contemplated for production herein may be used in compositions including, but not limited to, partially or wholly dehydrated or hydrated vaccine formulations or other viral formulations.

In certain embodiments of the invention as defined in the claims, the virus may be a live attenuated virus and may include, but is not limited to, one or more live, attenuated flavivirus vaccines, including but not limited to, attenuated yellow fever viruses (*e.g*. 17D), attenuated Japanese encephalitis viruses, (*e.g.* SA 14-14-2), attenuated dengue viruses (*e.g.* DEN-2/PDK-53 or DEN-4Δ30), attenuated chimeric West Nile vaccines, or recombinant chimeric flaviviruses.

The kits for growing viral cultures of the invention as defined in the claims may include partially or wholly dehydrated viral cultures of use in generating live, attenuated virus populations for vaccine production or other viral composition uses. It is also contemplated that a kit may include one or more growth inducing compositions disclosed herein.

### Brief Description of the Drawings

The following drawings form part of the present specification and are included to further demonstrate certain embodiments herein of the invention as defined in the claims. The embodiments of the invention as defined in the claims may be better understood by reference to one or more of these drawings alone or in combination with the detailed description of specific embodiments thereof.
Fig. 1 represents an exemplary graph of pluronic effects on viral growth after cellular infection and introduction of a control agent or copolymer composition during viral adsorption.
Fig. 2 represents an exemplary graph illustrating growth of viral cultures in the presence of a copolymer during viral adsorption and/or growth.
Fig. 3 represents an exemplary graph illustrating growth of viral cultures in the presence of increasing amounts of a copolymer-containing composition during viral adsorption and growth.
Fig. 4 represents an exemplary graph illustrating growth of viral cultures in the presence or absence of a specific copolymer added during viral adsorption.
Fig. 5 represents an exemplary table illustrating change in plague size of exemplary viral cultures in the presence or absence of various concentrations of copolymer.

### Definitions

As used herein, "a" or "an" may mean one or more than one of an item.

As used herein, vessel can include, but is not limited to, test tube, mini- or microfuge tube, plate, tissue culture flask, cell factory, channel, vial, microtiter plate or container.

As used herein the specification, "subject" or "subjects" may include but are not limited mammals such as humans or mammals, domesticated or wild, for example dogs, cats, ferrets, rabbits, pigs, horses, cattle, or zoo animals.

As used herein, "about" can mean plus or minus ten percent.

As used herein, "high molecular weight surfactants" can mean a surface active, amphiphilic molecule greater than 1500 molecular weight.

As used herein, "EO-PO block copolymer" can mean a copolymer consisting of blocks of poly(ethylene oxide) and poly(propylene) oxide. In addition, as used herein, "Pluronic" can mean EO-PO block copolymers in the EOx-POy-EOx. This configuration of EO-PO block copolymer is also referred to as "Poloxamer" or "Synperonic".

As used herein, "attenuated virus" can mean a virus that demonstrates reduced or no clinical signs of viral-related disease when administered to a subject such as a mammal (*e.g.* a human or an animal).

As used herein, "accelerate" can mean decreasing the lag time before virus production begins or increasing the rate of viral production such that higher concentrations of virus are produced in a shorter amount of time or such that plaque size is increased in some embodiments relative to a control.

As used herein, "killed virus vaccine" can mean a vaccine prepared by inactivating a virus by any of a number of physical or chemical means known in the art.

### Description

In the following sections, various exemplary compositions and methods of the invention as defined in the claims are described in order to detail various embodiments thereof. It will be obvious to one skilled in the art that practicing the various embodiments of the invention as defined in the claims does not require the employment of all or even some of the details outlined herein, but rather that concentrations, times and other details may be modified through routine experimentation. In some cases, well-known methods or components have not been included in the description.

Embodiments of the invention as defined in the claims herein concern using various compositions to enhance growth rate or reduce lag time of viral growth in a culture. In accordance with these embodiments, compositions can include copolymer agents. Aspects of the invention as defined in the claims generally relate to methods, compositions and uses for inducing and accelerating viral growth. In certain aspects, the invention as defined in the claims relates generally to methods, compositions and uses of copolymer compositions for accelerating viral growth and/or increasing viral plaque size. In other embodiments, methods, compositions and uses of copolymer compositions for accelerating flaviviral growth, reducing lag in growth and/or increasing plaque size. Certain copolymer compositions contemplated herein include, but are not limited to, Pluronic F127, Pluronic F68, Pluronic P85, Pluronic P123, other EO-PO block copolymers of greater than 3,000-4,000 MW or combinations thereof.

### Copolymers

In certain embodiments the invention as defined in the claims, compositions can include copolymers, for example, pluronic F127. Pluronic F127 (also referred to herein as F127) is a non-ionic polyoxyethylene-poloxypropylene copolymer. Pluronic block copolymers are known under their non-proprietary name as poloxamers. They were initially developed for use as surfactants. These compounds consist of hydrophilic ethylene oxide (EO) and hydrophobic propylene oxide (PO) blocks. The EO-PO block copolymers can include blocks of polyethylene oxide (-CH2CH2O- designated EO) and polypropylene oxide (-CH2CHCH3O- designated PO). The PO block can be flanked by two EO blocks in an EOx-POy-EOx arrangement. Since the PO component is hydrophilic and the EO component is hydrophobic, overall hydrophilicity, molecular weight and the surfactant properties can be adjusted by varying x and y in the EOx-POy-EOx block structure. According to the manufacturer, (e.g. BASF, Lutrol®F127) F127 can be used as a thickening agent and co-emulsifier in creams and liquid emulsions.

F127 undergoes a process known as reverse thermogelation, as it undergoes a phase transition from liquid to a gel upon reaching physiological temperatures. Higher temperatures promote the dehydration of an alkylene oxide unit of the block polymer and this can result in decreased solubility. Specifically, at high concentrations (for example: about 10% w/v) certain types of the higher molecular weight EO-PO block copolymers will undergo reverse gelation, forming a gel as the temperature increases. Additionally, when these block copolymers reside above the critical micelle concentration (CMC), they self assemble into micelles. In aqueous solutions, the EO-PO block copolymers will self-assemble into micelles with a PO core and a corona of hydrophilic EO groups. In certain studies, EO-PO block copolymer formulations have been investigated as potential drug delivery agents for a variety of hydrophobic drugs and for protein, DNA or inactivated vaccines.

The mechanism of activity of these pluronic block copolymers is currently unknown. Although, Pluronic F127 has been studied as a sustained release component of a vaccine delivery system in combination with chitosan. Vaccination of mice with Tetanus toxoid containing F127 increased the antibody response in intranasally delivered and systemically delivered tetanus antigens. In certain methods, pluronics have been shown to induce changes in the microviscosity and fluidity of cell membranes, which may contribute to its versatility.

Pluronic F127 has been used in a variety of human pharmaceutical applications including dental, oral and laxative pharmaceuticals. Vaccine formulations have also used surfactants as stabilizers to prevent material loss. Studies of DNA vaccine delivery with certain concentrations of F127 (0.01% w/v) have shown increased drug delivery, possibly by potentiating cellular uptake and recruitment of mature dendritic cells. Gel formation at body temperatures permits use of the EO-PO block copolymer gels to act as a drug depot in vaccine and drug delivery applications.

Certain compositions the invention as defined in the claims can include copolymers either alone or in combination with other agents or compounds. In addition, compositions disclosed herein may include a media composition having one or more copolymer agent(s) added to the media in addition to other media supplements. Medias of use in compositions disclosed herein may include any media known in the art known to grow viral organisms contemplated herein or a media specific for a particular viral organism. Other embodiments the invention as defined in the claims herein concern combinations of excipients that greatly enhance the growth of live viruses (e.g. attenuated viruses). Yet other compositions and methods the invention as defined in the claims are directed to reducing the lag time related to growth of viral organisms. Some embodiments the invention as defined in the claims concern modulating plague size of viral organisms. Copolymers of use herein include, but are not limited to, Pluronic F127, Pluronic F68, Pluronic P85, Pluronic P123, other EO-PO block copolymers of greater than 3,000-4,000 MW or combinations thereof.

Compositions the invention as defined in the claims may be used alone or in combination with media before, during, and/or after viral cultures have been introduced to host culture cell media of compositions disclosed herein may be liquid, solid or semi-solid liquid. In certain embodiments the invention as defined in the claims supplementary compositions may be added during entire viral growth periods in order to monitor, adjust or stimulate viral growth processes. In other embodiments the invention as defined in the claims, one or more supplementary copolymer compositions may be added to reduce lag time, accelerate viral growth and/or increase viral plaque size. Compositions the invention as defined in the claims may be used alone, in combination with other supplements (*e.g*. vitamins, metal ions and amino acids), or as a media supplement when media is added to the cultures.

Other embodiments of the invention as defined in the claims include stocks for culturing viral cultures such as live attenuated virus including, but not limited to, Picornaviruses (*e*.*g*., polio virus, foot and mouth disease virus), Caliciviruses (*e.g.,* SARS virus, feline infectious peritonitis virus), Togaviruses (*e.g.,* sindbis virus, the equine encephalitis viruses, chikungunya virus, rubella virus, Ross River virus, bovine diarrhea virus, hog cholera virus), Flaviviruses (*e*.*g*., dengue virus, West Nile virus, yellow fever virus, Japanese encephalitis virus, St. Louis encephalitis virus, tick-borne encephalitis virus), Coronaviruses (*e*.*g*., human coronaviruses (common cold), swine gastroenteritis virus), Rhabdoviruses (*e*.*g*., rabies virus, vesicular stomatitis viruses), Filoviruses (*e.g.,* Marburg virus, Ebola virus.), Paramyxoviruses (*e.g.,* measles virus, canine distemper virus, mumps virus, parainfluenza viruses, respiratory syncytial virus, Newcastle disease virus, rinderpest virus), Orthomyxoviruses (*e*.*g*., human influenza viruses, avian influenza viruses, equine influenza viruses), Bunyaviruses (*e*.*g*., hantavirus, LaCrosse virus, Rift Valley fever virus), Arenaviruses (*e.g*., Lassa virus, Machupo virus), Reoviruses (*e.g.,* human reoviruses, human rotavirus), Birnaviruses (*e*.*g*., infectious bursal virus, fish pancreatic necrosis virus), Retroviruses (*e*.*g*., HIV 1, HIV 2, HTLV-1, HTLV-2, bovine leukemia virus, feline immunodeficiency virus, feline sarcoma virus, mouse mammary tumor virus), Hepadnaviruses (*e*.*g*., hepatitis B virus.), Parvoviruses (human parvovirus B, canine parvovirus, feline panleukopenia virus) Papovaviruses (*e.g.,* human papillomaviruses, SV40, bovine papillomaviruses), Adenoviruses (*e.g*., human adenovirus, canine adenovirus, bovine adenovirus, porcine adenovirus), Herpes viruses (*e.g*., herpes simplex viruses, varicella-zoster virus, infectious bovine rhinotracheitis virus, human cytomegalovirus, human herpesvirus 6), and Poxviruses (*e.g*., vaccinia, fowlpoxviruses, raccoon poxvirus, skunkpox virus, monkeypoxvirus, cowpox virus, musculum contagiosum virus).

In accordance with these embodiments, certain live attenuated viruses include, but are not limited to, live, attenuated flaviviruses. Some embodiments of the invention as defined in the claims can include, but are not limited to, one or more live, attenuated viruses, such as one or more live, attenuated flaviviruses grown in one or more copolymer compositions alone or in combination with other agents. In accordance with these embodiments, a flavivirus can include, but are not limited to, dengue virus, West Nile virus, yellow fever virus, Japanese encephalitis virus, St. Louis encephalitis virus, tick-borne encephalitis virus or other known flavivirus.

In other embodiments of the invention as defined in the claims, compositions and methods can increase plaque size in reduced or similar time periods of growth, compared to controls not grown compositions disclosed herein, for use in assessing viral activity or tittering viral preparations. Alternatively, such compositions and methods can reduce lag time or accelerate growth time for up to several days earlier than control viral cultures not using compositions contemplated herein. In certain embodiments of the invention as defined in the claims predetermined viral titers may occur several hours, a half a day, 1 day, 2 days, 3 days, 4 days or even up to 10 days earlier than virus preparations grown in other media known in the art or supplemental compositions furnished to cultures having no copolymer. Optimal viral titer of some embodiments may be about 1x10⁶ pfu/mL to about 1x10⁸ pfu/mL. In certain embodiments of the invention as defined in the claims, a flaviviral titer may reach concentrations of about 1x10⁷ pfu/mL in about 4 days in media containing F127, as compared to cultures grown in media without F127 which takes about 6 days.

Some embodiments of the invention as defined in the claims concern compositions and methods for modulating time for growth of a viral culture to reach a predetermined concentration. In accordance with these embodiments, time for growth may be reduced by about 5%, about 10%, about 15%, about 20%, about 25%, about 30%, about 35%, about 40% and more. In various embodiments of the invention as defined in the claims a predetermined viral culture density may be accomplished in about 80%, or about 70%, or about 60% of time using compositions disclosed herein compared to other compositions known in the art.

In certain embodiments of the invention as defined in the claims, viral cultures contemplated for production herein may be used in compositions including, but not limited to, partially or wholly dehydrated or hydrated vaccine formulations. In other embodiments of the invention as defined in the claims, viral cultures contemplated herein for production of vaccine formulations can be cultured for reduced time and costs. In addition, production of these vaccine formulations can be reduced in labor, time and costs, for example, in times when an epidemic or outbreak of flaviviral-associated diseases occur and vaccine formulations are required in a short period of time.

In some embodiments of the invention as defined in the claims, a live attenuated virus for use in a vaccine composition contemplated herein may include, but is not limited to, one or more live, attenuated flavivirus vaccines, including but not limited to, attenuated yellow fever viruses (such as 17D), attenuated Japanese encephalitis viruses, (such as SA 14-14-2), attenuated dengue viruses (such as DEN-2/PDK-53 or DEN-4Δ30), attenuated chimeric West Nile virus, or recombinant chimeric flaviviruses. In certain embodiments, the flaviviral cultures of use in a vaccine composition can be grown in media compositions having one or more copolymer disclosed herein.

Other embodiments of the invention as defined in the claims concern virus populations of use in formulations and methods directed to vaccine formulations capable of reducing or preventing onset of a medical condition caused by one or more of the flaviviruses contemplated herein. In accordance with these embodiments, medical conditions may include, but are not limited to, West Nile infection, dengue fever, Japanese encephalitis, Kyasanur forest disease, Murray valley encephalitis, Alkhurma hemorrhagic fever, St. Louis encephalitis, tick-borne encephalitis, yellow fever and hepatitis C virus infection. Thus, production time for generating these formulations can be reduced using compositions of the invention as defined in the claims for increasing growth, reducing lag time and/or increasing plague size of viral populations used in formulations disclosed.

Other embodiments of the invention as defined in the claims concern virus compositions of use in therapeutic applications. Such uses may include, but are not limited to, gene therapy applications. Viruses used to deliver genes to cells in gene therapy applications include lentiviruses, adenoviruses, adeno-associated viruses, and herpesviruses. Other uses of virus compositions may include, but are not limited to, cancer virus therapies *(e.g.,* "oncolytic" viruses) or cancer immunotherapies.

It is contemplated herein that any media used for growth of cell cultures (e.g. host cells) may be of use herein. For example, commonly used medias for cell cultures are contemplated. In accordance with these embodiments, media may include, but are not limited to DMEM (Dulbecco's Modified Eagle Medium, high glucose, with L-glutamine, with pyridoxine hydrochloride, without sodium pyruvate containing 3.7 g sodium bicarbonate per liter), MEM, BSS/YE-LAH, F-10 (Ham's), F-12, M-199, RPMI, Agars, LB Broth, and PBS- based medias. In addition, it is contemplated that cells may be cultured by any means known in the art. For example, cells may be grown in confluent layers, as suspensions, in multiple layers, in roller bottles, in wells or in tubes.

In certain embodiments of the invention as defined in the claims, host cells can be used to culture viruses disclosed herein. Any cell known to host viruses disclosed herein is contemplated. Some host cells of use for growing viruses disclosed herein include, but are not limited to, Vero (African green monkey Vero cells), LLC-MK₂ cells, C6/36 mosquito cells or other cells known in the art.

In some embodiments of the invention as defined in the claims compositions have one or more high molecular weight surfactants or copolymer compounds of use in methods for culturing various viral cultures where some compositions disclosed herein are capable of modulating various aspects of viral growth (e.g. larger plaque size, reduced lag phase) by about 10%, by about 15%, by about 20%, by about 25%, by about 30%, by about 35%, by about 40%, by about 45%, by about 50% or more, compared to compositions not having a copolymer composition.

### Kits

Further embodiments of the invention as defined in the claims concern kits of use for methods and compositions described herein. Compositions including, but not limited to, copolymer compositions and live virus formulations may be provided in a kit. Kits can also include, but are not limited to, a suitable container, copolymer compositions, live virus compositions detailed herein and optionally, one or more additional agents such as other anti-viral agents, anti-fungal agents or anti-bacterial agents for example, to modulate growth of undesireable species.

The kits may further include a suitably aliquoted copolymer composition of use for viral cultures. In addition, compositions herein may be partially or wholly dehydrated or aqueous viral cultures and/or host cells for propagating the viruses as well as liquid or partially or wholly dehydrated medias. Kits contemplated herein may be stored at room temperatures, frozen or at refrigerated temperatures as disclosed herein depending on the particular formulations and components.

The container means of the kits will generally include at least one vial, test tube, flask, bottle, syringe or other container means, into which a composition may be placed, and preferably, suitably aliquoted. Where an additional component is provided, the kit will also generally contain one or more additional containers into which this agent or component may be placed. Kits herein will also typically include a means for containing the agent, composition and any other reagent containers in close confinement for commercial sale. Such containers may include injection or blow-molded plastic containers into which the desired vials are retained.

The following examples are included to demonstrate certain embodiments presented herein. It should be appreciated by those of skill in the art that the techniques disclosed in the examples which follow represent techniques discovered to function well in the practices disclosed herein, and thus can be considered for its practice.

### EXAMPLES

### Example 1

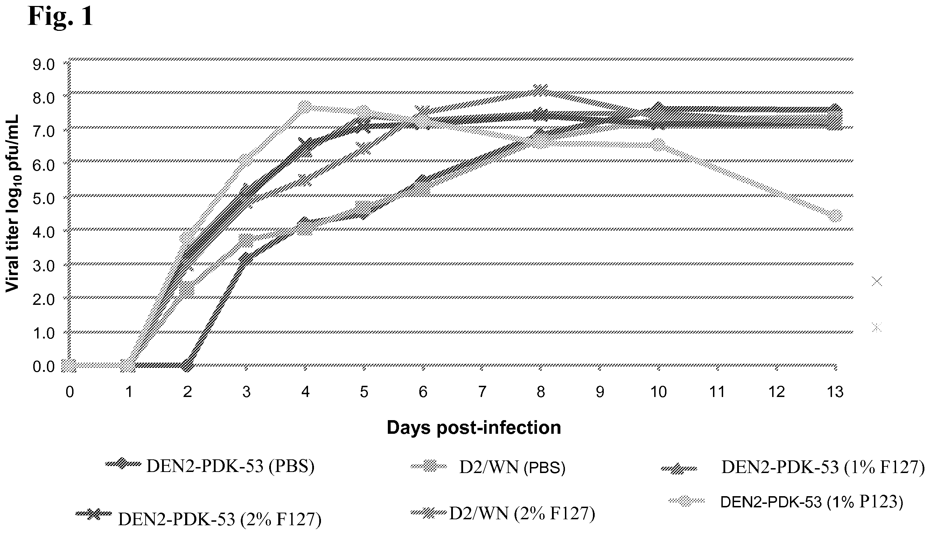

In one exemplary method, represented in Fig. 1, pluronic effects on flavivirus growth were examined in one exemplary cell line, Vero cells (African green monkey Vero cells). Vero cells were grown to confluency for example, in T-75 cm2 flasks 2 days prior to infection with flavivirus (as indicated) at an MOI of 0.001. Virus adsorption for 180 minutes was assessed in 2 mL PBS in the presence or absence of pluronic (P123 or F127). Control samples contained viral adsorption in PBS without a copolymer. Growth media (18 mL serum-free DMEM) was added after adsorption. Aliquots were taken daily, and titrated on Vero cell mono layers. Viral titers were measured as illustrated in Fig. 1.

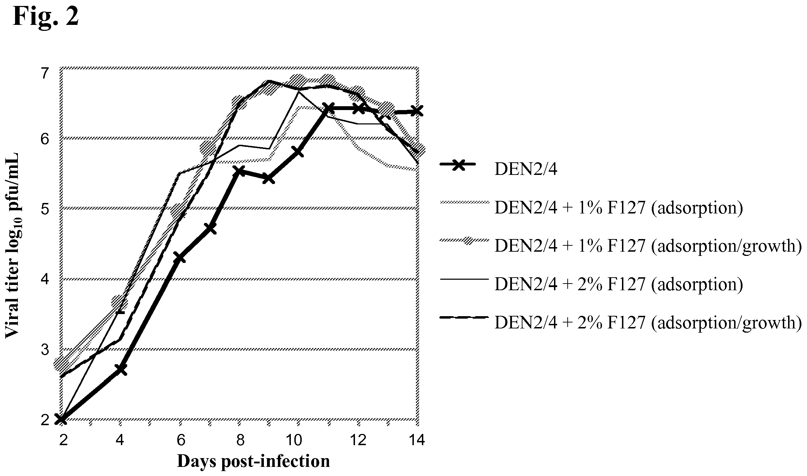

In another example, illustrated in Fig. 2, growth of the chimeric flavivirus DEN-2/4 in Vero cells, without or with varying concentrations of copolymer, Pluoronic F127 was examined. Vero cells were grown to confluency for example, in T-75cm2 flasks 2 days prior to infection with DEN-2/4 at an MOI of 0.001. Virus was adsorbed for 120 minutes in 2 mL DMEM with or without F127. Viral inoculums were rinsed from the cell monolayer with PBS followed by addition of 25 mL indicated growth medium containing 5% FBS with or without F127 and grown for 14 days. Aliquots were taken daily, and titrated on vero cell monolayers. Viral titers were measured as illustrated in Fig.2.

### Example 2

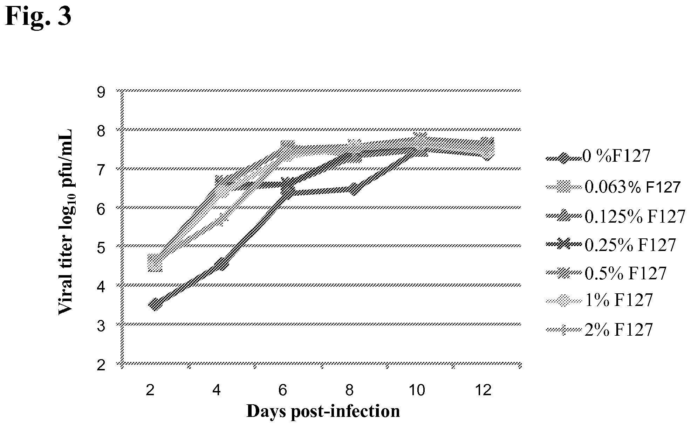

In another exemplary method, growth of DEN 2/4 (Dengue 2/4) chimera in Vero cells containing increasing amounts of F127 during adsorption and growth were examined (see for example, Fig. 3). A confluent monolayer of Vero cells grown in T75cm2 flasks in 25 mL DMEM medium containing 10% FBS and control or increasing concentrations of F127. Exemplary F127 concentrations used in this experiment included 0.063%, 0.125%, 0.25%, 0.5%, 1.0% and 2.0% F127. The cells were infected with DEN2/4 at MOI = 0.001. Parameters included adsorption for 1.5 hours in 1mL DMEM/F127. Aliquots were taken every other day, and titrated on Vero cell monolayers. Viral titers were measured as illustrated in Fig.3.

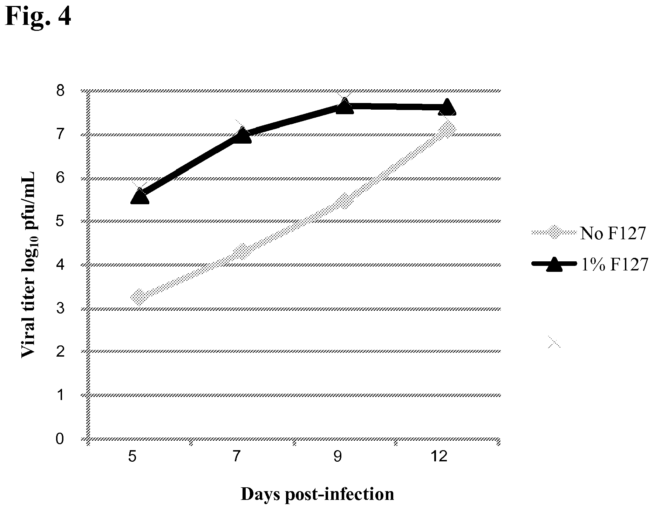

In addition, another experiment analyzed effects of the copolymer F127 during viral adsorption of the chimeric flavivirus DEN2/1. In this example, DEN2/1 was adsorbed onto a confluent flask of Vero cells at an MOI of 0.001 for 90 minutes. Adsorption was performed in 1 mL of growth media (BA-1) with or without 1% F127. After viral adsorption, 20 mL DMEM containing 2% FBS and no F127 was added to the cultures. Aliquots were taken every other day and titrated on Vero cell monolayers. Viral titers can be measured as illustrated in Fig. 4.

### Example 3

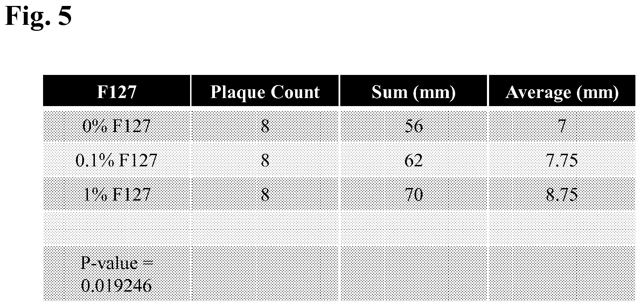

Another exemplary method analyzed whether or not plaque size increased in the presence of an exemplary copolymer, F127. Fig. 5 represents an exemplary table, Table 1. This experiment demonstrated that flavivirus plaque size increases in the presence of increasing concentrations of pluronic F127 during growth. Here, confluent monolayer of Vero cells were grown in T75cm2 flask in 20 mL DMEM2% FBS medium in the presence or absence of F127 where the Vero cells were infected with DEN2/4 at MOI = 0.001. Adsorption was 1.5 hours and 1mL DMEM in the presence (0.1% or 1.0%) or absence of F127. Plaques (*e.g.* 8) were visualized on a light box, and their diameter was measured. Table 1 represents results of one way ANOVA of plaque size (mm) differences in DENVax 2/4 growth in the absence of F127 or presence of increasing concentrations of F127. Materials and Methods:

It is contemplated that any method known in the art can be used for any composition, method and/or uses described herein. In certain embodiments, it is contemplated that certain methods will be more suited for viral growth, for example materials and methods of use for flaviviral growth, than other methods. In other embodiments, it is contemplated that certain methods will be more suited for the growth of Dengue, than suited for other flaviviruses. The following provides a brief description as to the methods to grow a high-titer chimeric Dengue vaccine or any live-attenuated flavivirus in the presence of F127. Using T-75cm2 flask, for example, Vero cells are seeded 2 days prior to viral infection/adsorption at a density of 5 x 10^6 cells per flask. This viral growth can be "scaled up" to include tissue culturing vessels ranging from T-25cm2 to 10-stack cell factories. Virus is adsorbed onto a confluent monolayer of Vero cells 2 days after cell seeding in 1mL DMEM containing F127 (0.1%). The culture vessels are incubated for 1.5 hours at 37°C with rocking of the vessel every 10 minutes. After viral adsorption, the cell monolayers are washed three times with 10mL PBS. Growth medium (10 mL DMEM pH=7.2, containing 3.7 g/L NaHCO₃ and 0.1% F127 with no FBS) is then added to the monolayers, and incubated with or without aeration at 37°C for 4 days. On day 4 of viral growth, the growth medium is replaced, as done after viral adsorption. Starting at day 6, and until day 12, the infectious medium is completely removed from the growth chamber and centrifuged for clarification. This viral growth is stabilized, and stored at -80°C until its titer can be examined by plaque assay on Vero cell monolayers. After daily harvests, the growth medium is replaced on the tissue culture vessels as done on day 4. Daily harvests continue until day 12. Daily harvests are individually titered on Vero cells, and high-titer harvests can be pooled to obtain a homogeneous sample. Often, the first day harvest (day 6) is not included, to avoid high levels of host-cell (Vero) DNA.

**Table 1. Example of DMEM - F12 : F-12 Nutrient Mixture (Ham), powder (21700) with L-glutamine Additives per 10 L: 11.76 g Sodium Bicarbonate, 100 ml Penicillin Streptomycin Adjust the pH to 7.2**

| **COMPONENTS** | **Mole. Weight** | **Conc. (mg/L)** | **Molarity (mm)** |
|---|---|---|---|
| **INORGANIC SALTS:** | | | |
| Calcium chloride (Anhydrous) | 111 | 33.22 | 0.299 |
| Cupric sulfate (CuSO4-5H2O) | 250 | 0.0025 | 0.00001 |
| Ferric sulfate (FeSO4-7H2O) | 278 | 0.834 | 0.003 |
| Potassium chloride (KCl) | 75 | 223.60 | 2.98 |
| Magnesium chloride (Anhydrous) | 95 | 57.22 | 0.60 |
| Sodium chloride (NaCl) | 58 | 7599.00 | 131.00 |
| Sodium bicarbonate (NaHCO3) | 84 | 1176.00 | 14.00 |
| Sodium phosphate, dibas (Anhydrous) | 142 | 142.00 | 1.00 |
| Zinc sulfate (ZnSO4-7H2O) | 288 | 0.863 | 0.003 |
| | | | |

| **OTHER COMPOUNDS:** | | | |
|---|---|---|---|
| D-Glucose | 180 | 1802.00 | 1.00 |
| Hypoxanthine Na | 159 | 4.77 | 0.03 |
| Linoleic Acid | 280 | 0.084 | 0.0003 |
| Lipoic Acid | 206 | 0.21 | 0.000971 |
| Phenol red | 398 | 1.20 | 0.003 |
| Putrescine-2HCl | 161 | 0.161 | 0.001 |
| Sodium Pyruvate | 110 | 110.00 | 1.00 |
| Thymidine | 242 | 0.70 | 0.003 |
| | | | |

| **AMINO ACIDS:** | | | |
|---|---|---|---|
| L-Alanine | 89 | 8.90 | 0.100 |
| L-Arginine hydrochloride | 211 | 211.00 | 1.00 |
| L-Asparagine-H2O | 150 | 15.01 | 0.100 |
| L-Aspartic acid | 133 | 13.30 | 0.100 |
| L-Cysteine-HCl-H2O | 176 | 35.12 | 0.200 |
| L-Glutamic acid | 147 | 14.70 | 0.100 |
| L-Glutamine | 146 | 146.00 | 1.00 |
| Glycine | 75 | 7.50 | 0.100 |
| L-Histidine-HCl-H2O | 210 | 21.00 | 0.0998 |
| L-Isoleucine | 131 | 4.00 | 0.030 |
| L-Leucine | 131 | 13.10 | 0.100 |
| L-Lysine hydrochloride | 183 | 36.50 | 0.199 |
| L-Methionine | 149 | 4.50 | 0.030 |
| L-Phenylalanine | 165 | 5.00 | 0.030 |
| L-Proline | 115 | 34.50 | 0.300 |
| L-Serine | 105 | 10.50 | 0.100 |
| L-Threonine | 119 | 11.90 | 0.100 |
| L-Tryptophan | 204 | 2.04 | 0.010 |
| L-Tyrosine 2Na 2H2O | 225 | 7.81 | 0.03 |
| L-Valine | 117 | 11.70 | 0.100 |
| | | | |

| **VITAMINS:** | | | |
|---|---|---|---|
| Biotin | 244 | 0.0073 | 0.00003 |
| D-Calcium pantothenate | 477 | 0.50 | 0.001 |
| Choline chloride | 140 | 14.00 | 0.0997 |
| Folic acid | 441 | 1.30 | 0.0029 |
| i-Inositol | 180 | 18.00 | 0.100 |
| Niacinamide | 122 | 0.036 | 0.0003 |
| Pyridoxine hydrochloride | 206 | 0.06 | 0.0003 |
| Riboflavin | 376 | 0.037 | 0.000101 |
| Thiamine hydrochloride | 337 | 0.30 | 0.001 |
| Vitamin B12 | 1,355 | 1.40 | 0.001 |

## Claims

1. A composition for growing viral cultures comprising:
one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v);
a viral culture;
a host cell; and
a medium for growing the viral culture,
wherein the EO-PO block copolymer accelerates growth of viral cultures.

2. The composition according to claim 1, wherein the viral cultures are selected from the group consisting of Flavivirus, Togavirus, Coronavirus, Rhabdovirus, Filovirus, Paramyxovirus, Orthomyxovirus, Bunyavirus, Arenavirus, Retrovirus, Hepadnavirus, Pestivirus, Picornavirus, Calicivirus, Reovirus, Parvovirus, Papovavirus, Adenovirus, Herpes virus, and Poxvirus.

3. The composition according to claim 1, wherein the viral cultures are flavivirus cultures or poxvirus cultures.

4. The composition according to any one of claims 1 to 3, wherein the viral cultures are dengue virus cultures.

5. The composition according to any one of claims 1 to 4, wherein at least one of the EO-PO block copolymer is poloxamer 407 (Pluronic F127) and its concentration is about 0.063 % to about 2.0 % (w/v).

6. The composition of claim 1, wherein the medium comprises Dulbecco's Modified Eagle Medium (DMEM).

7. The composition of claim 1, wherein the viral culture comprises flavivirus and the host cell culture comprises Vero (African green monkey Vero cells), LLC-MK2 cells (monkey kidney cells) or C6/36 mosquito cells.

8. A method for increasing viral growth rate comprising, administering to a host cell culture infected with a virus, a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), and wherein the composition increases viral growth rate.

9. A method for increasing viral growth rate comprising, administering a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001% to about 3.0 % (w/v), to a host cell culture, before, during, or after viral infection of the host cell culture.

10. A method for increasing plaque size of a viral culture comprising, administering to a host cell culture infected with a virus, a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), and wherein the composition increases viral plaque size compared to a control viral culture without administering one or more EO-PO copolymers.

11. A method for reducing growth lag time of a viral culture comprising, administering to a host cell culture infected with a virus, a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v), and wherein the composition reduces lag time of the viral cultures compared to a control viral culture without administering one or more EO-PO block copolymers.

12. The method of claim 11, wherein the viral cultures are flaviviral cultures.

13. A kit for culturing viruses comprising;
at least one container;
a composition comprising one or more ethylene oxide propylene oxide (EO-PO) block copolymers, wherein the EO-PO block copolymers comprise poloxamer 407 (Pluronic F127), poloxamer 403 (Pluronic P123) or a combination thereof, wherein the concentration of EO-PO block copolymer is about 0.001 % to about 3.0 % (w/v); and
one or more viral cultures,
wherein the EO-PO block copolymer accelerates growth of viral cultures.

14. The kit of claim 13, wherein the EO-PO block copolymer is poloxamer 407 (Pluronic F127) and the concentration of the EO-PO block copolymer is about 0.063 % to about 2.0 % (w/v).

15. The kit of claim 13, wherein the one or more viral cultures comprise one or more flavivirus cultures.

16. The kit of claim 13, wherein the one or more viral cultures comprise one or more dengue virus cultures.

## Patentansprüche

1. Zusammensetzung für das Anwachsen von Viruskulturen, umfassend:
ein oder mehrere Ethylenoxid-Propylenoxid (EO-PO)-Blockcopolymere, wobei die EO-PO-Blockcopolymere Poloxamer 407 (Pluronic F127), Poloxamer 403 (Pluronic P123) oder eine Kombination davon umfassen, wobei die Konzentration des EO-PO-Blockcopolymers etwa 0,001% bis etwa 3,0% (Gew./Vol.) beträgt;
eine Viruskultur;
eine Wirtszelle; und
ein Medium für das Anwachsen der Viruskultur,
wobei das EO-PO-Blockcopolymer das Wachstum von Viruskulturen beschleunigt.

2. Zusammensetzung nach Anspruch 1, wobei die Virenkulturen ausgewählt sind aus der Gruppe bestehend aus Flavivirus, Togavirus, Coronavirus, Rhabdovirus, Filovirus, Paramyxovirus, Orthomyxovirus, Bunyavirus, Arenavirus, Retrovirus, Hepadnavirus, Pestivirus, Picornavirus, Calicivirus, Reovirus, Parvovirus, Papovavirus, Adenovirus, Herpesvirus und Pockenvirus.

3. Zusammensetzung nach Anspruch 1, wobei die Viruskulturen Flavivirus-Kulturen oder Pockenvirus-Kulturen sind.

4. Zusammensetzung nach einem der Ansprüche 1 bis 3, wobei die Viruskulturen Dengue-Virus-Kulturen sind.

5. Zusammensetzung nach einem der Ansprüche 1 bis 4, wobei mindestens eines der EO-PO-Blockcopolymere Poloxamer 407 (Pluronic F-127) ist und dessen Konzentration etwa 0,063% bis etwa 2,0% (Gew./Vol.) beträgt.

6. Zusammensetzung nach Anspruch 1, wobei das Medium Dulbecco's Modified Eagle Medium (DMEM) umfasst.

7. Zusammensetzung nach Anspruch 1, wobei die Viruskultur Flavivirus umfasst und die Wirtszellkultur Vero-Zellen (Grüne Meerkatzen Vero-Zellen), LLC-MK2-Zellen (Rhesusaffen-Nierenzellen) oder C6/36-Stechmückenzellen umfasst.

8. Verfahren zum Erhöhen der Wachstumsrate von Viren umfassend das Verabreichen einer Zusammensetzung umfassend ein oder mehrere Ethylenoxid-Propylenoxid (EO-PO)-Blockcopolymere an eine, mit einem Virus infizierte Wirtszellkultur, wobei die EO-PO-Blockcopolymere Poloxamer 407 (Pluronic F127), Poloxamer 403 (Pluronic P123) oder eine Kombination davon umfassen, wobei die Konzentration des EO-PO-Blockcopolymers etwa 0,001% bis etwa 3,0% (Gew./Vol.) beträgt und wobei die Zusammensetzung die Wachstumsrate von Viren erhöht.

9. Verfahren zum Erhöhen der Wachstumsrate von Viren umfassend das Verabreichen einer Zusammensetzung umfassend ein oder mehrere Ethylenoxid-Propylenoxid (EO-PO)-Blockcopolymere, wobei die EO-PO-Blockcopolymere Poloxamer 407 (Pluronic F127), Poloxamer 403 (Pluronic P123) oder eine Kombination davon umfassen, wobei die Konzentration des EO-PO-Blockcopolymers etwa 0,001% bis etwa 3,0% (Gew./Vol.) beträgt, an eine Wirtszellkultur vor, während oder nach Virusinfektion der Wirtszellkultur.

10. Verfahren zur Erhöhung der Plaque-Größe einer Viruskultur umfassend das Verabreichen einer Zusammensetzung umfassend ein oder mehrere Ethylenoxid-Propylenoxid (EO-PO)-Blockcopolymere an eine, mit einem Virus infizierte Wirtszellkultur, wobei die EO-PO-Blockcopolymere Poloxamer 407 (Pluronic F127), Poloxamer 403 (Pluronic P123) oder eine Kombination davon umfassen, wobei die Konzentration des EO-PO-Blockcopolymers etwa 0,001% bis etwa 3,0% (Gew./Vol.) beträgt, und wobei die Zusammensetzung die Plaque-Größe der Viren im Vergleich zu einer Virus-Kontrollkultur ohne Verabreichung eines oder mehrerer EO-PO-Copolymere erhöht.

11. Verfahren zum Verringern der Wachstumsverzögerungszeit einer Viruskultur umfassend das Verabreichen einer Zusammensetzung umfassend ein oder mehrere Ethylenoxid-Propylenoxid (EO-PO)-Blockcopolymere an eine, mit einem Virus infizierte Wirtszellenkultur, wobei die EO-PO-Blockcopolymere Poloxamer 407 (Pluronic F127), Poloxamer 403 (Pluronic P123) oder eine Kombination davon umfassen, wobei die Konzentration des EO-PO-Blockcopolymers etwa 0,001% bis etwa 3,0% (Gew./Vol.) beträgt und wobei die Zusammensetzung die Verzögerungszeit der Viruskulturen im Vergleich zu einer Kontrollviruskultur ohne Verabreichung eines oder mehrerer EO-PO-Blockcopolymere verringert.

12. Verfahren nach Anspruch 11, wobei die Viruskulturen Flavivirus-Kulturen sind.

13. Kit zum Kultivieren von Viren, umfassend:
mindestens ein Behältnis;
eine Zusammensetzung umfassend ein oder mehrere Ethylenoxid-Propylenoxid (EO-PO)-Blockcopolymere, wobei die EO-PO-Blockcopolymere Poloxamer 407 (Pluronic F 127), Poloxamer 403 (Pluronic P123) oder eine Kombination davon umfassen, wobei die Konzentration von EO-PO-Blockcopolymer etwa 0,001% bis etwa 3,0% (Gew./Vol.) beträgt; und
eine oder mehrere Viruskulturen,
wobei das EO-PO-Blockcopolymer das Wachstum von Viruskulturen beschleunigt.

14. Kit nach Anspruch 13, wobei das EO-PO-Blockcopolymer Poloxamer 407 (Pluronic F-127) ist und die Konzentration des EO-PO-Blockcopolymers etwa 0,063% bis etwa 2,0% (Gew./Vol.) beträgt.

15. Kit nach Anspruch 13, wobei die eine oder mehreren Viruskulturen eine oder mehrere Flavivirus-Kulturen umfassen.

16. Kit nach Anspruch 13, wobei die eine oder mehreren Viruskulturen eine oder mehrere Dengue-Virus-Kulturen umfassen.

## Revendications

1. Composition pour la croissance de cultures virales comprenant :
un ou plusieurs copolymères blocs d'oxyde d'éthylène et d'oxyde de propylène (EO-PO), sachant que les copolymères blocs EO-PO comprennent du poloxamère 407 (Pluronic F127), du poloxamère 403 (Pluronic P123) ou une combinaison de ceux-ci, sachant que la concentration de copolymère bloc EO-PO est d'environ 0,001 % à environ 3,0 % (p/v) ;
une culture virale ;
une cellule hôte ; et
un milieu pour la croissance de la culture virale,
sachant que le copolymère bloc EO-PO accélère la croissance de cultures virales.

2. La composition selon la revendication 1, sachant que les cultures virales sont sélectionnées dans le groupe constitué par Flavivirus, Togavirus, Coronavirus, Rhabdovirus, Filovirus, Paramyxovirus, Orthomyxovirus, Bunyavirus, Arenavirus, Retrovirus, Hepadnavirus, Pestivirus, Picornavirus, Calicivirus, Reovirus, Parvovirus, Papovavirus, Adenovirus, Herpes virus, et Poxvirus.

3. La composition selon la revendication 1, sachant que les cultures virales sont des cultures de flavivirus ou des cultures de poxvirus.

4. La composition selon l'une quelconque des revendications 1 à 3, sachant que les cultures virales sont des cultures de virus de la dengue.

5. La composition selon l'une quelconque des revendications 1 à 4, sachant qu'au moins un du copolymère bloc EO-PO est du poloxamère 407 (Pluronic F-127) et sa concentration est d'environ 0,063 % à environ 2,0 % (p/v).

6. La composition de la revendication 1, sachant que le milieu comprend un milieu Eagle modifié de Dulbecco (DMEM).

7. La composition de la revendication 1, sachant que la culture virale comprend du flavivirus et la culture de cellule hôte comprend des cellules Vero (cellules Vero de singe vert africain), des cellules LLC-MK2 (cellules rénales de singe) ou des cellules de moustique C6/36.

8. Procédé d'augmentation du taux de croissance virale comprenant l'administration à une culture de cellule hôte infectée par un virus d'une composition comprenant un ou plusieurs copolymères blocs d'oxyde d'éthylène et d'oxyde de propylène (EO-PO), sachant que les copolymères blocs EO-PO comprennent du poloxamère 407 (Pluronic F127), du poloxamère 403 (Pluronic P123) ou une combinaison de ceux-ci, sachant que la concentration de copolymère bloc EO-PO est d'environ 0,001 % à environ 3,0 % (p/v), et sachant que la composition augmente le taux de croissance virale.

9. Procédé d'augmentation du taux de croissance virale comprenant l'administration d'une composition comprenant un ou plusieurs copolymères blocs d'oxyde d'éthylène et d'oxyde de propylène (EO-PO), sachant que les copolymères blocs EO-PO comprennent du poloxamère 407 (Pluronic F127), du poloxamère 403 (Pluronic P123) ou une combinaison de ceux-ci, sachant que la concentration de copolymère bloc EO-PO est d'environ 0,001 % à environ 3,0 % (p/v), à une culture de cellule hôte, avant, pendant, ou après une infection virale de la culture de cellule hôte.

10. Procédé d'augmentation de la taille de plaque d'une culture virale comprenant l'administration à une culture de cellule hôte infectée par un virus d'une composition comprenant un ou plusieurs copolymères blocs d'oxyde d'éthylène et d'oxyde de propylène (EO-PO), sachant que les copolymères blocs EO-PO comprennent du poloxamère 407 (Pluronic F127), du poloxamère 403 (Pluronic P123) ou une combinaison de ceux-ci, sachant que la concentration de copolymère bloc EO-PO est d'environ 0,001 % à environ 3,0 % (p/v), et sachant que la composition augmente la taille de plaque virale en comparaison à une culture virale de contrôle sans administration d'un ou de plusieurs copolymères EO-PO.

11. Procédé de réduction d'un temps de latence de la croissance d'une culture virale comprenant l'administration à une culture de cellule hôte infectée par un virus d'une composition comprenant un ou plusieurs copolymères blocs d'oxyde d'éthylène et d'oxyde de propylène (EO-PO), sachant que les copolymères blocs EO-PO comprennent du poloxamère 407 (Pluronic F127), du poloxamère 403 (Pluronic P123) ou une combinaison de ceux-ci, sachant que la concentration de copolymère bloc EO-PO est d'environ 0,001 % à environ 3,0 % (p/v), et sachant que la composition réduit le temps de latence des cultures virales en comparaison à une culture virale de contrôle sans administration d'un ou de plusieurs copolymères blocs EO-PO.

12. Le procédé de la revendication 11, sachant que les cultures virales sont des cultures flavivirales.

13. Kit pour la mise en culture de virus comprenant :
au moins un récipient ;
une composition comprenant un ou plusieurs copolymères blocs d'oxyde d'éthylène et d'oxyde de propylène (EO-PO), sachant que les copolymères blocs EO-PO comprennent du poloxamère 407 (Pluronic F127), du poloxamère 403 (Pluronic P123) ou une combinaison de ceux-ci, sachant que la concentration de copolymère bloc EO-PO est d'environ 0,001 % à environ 3,0 % (p/v) ; et
une ou plusieurs cultures virales,
sachant que le copolymère bloc EO-PO accélère la croissance de cultures virales.

14. Le kit de la revendication 13, sachant que le copolymère bloc EO-PO est du poloxamère 407 (Pluronic F-127) et la concentration du copolymère bloc EO-PO est d'environ 0,063 % à environ 2,0 % (p/v).

15. Le kit de la revendication 13, sachant que l'une ou les plusieurs cultures virales comprennent une ou plusieurs cultures de flavivirus.

16. Le kit de la revendication 13, sachant que l'une ou les plusieurs cultures virales comprennent une ou plusieurs cultures de virus de la dengue.
